# EUROPEAN PATENT SPECIFICATION

(11) **EP 1 173 453 B1**
(45) Date of publication and mention of the grant of the patent: **30.06.2004**
(21) Application number: 00920768.9
(22) Date of filing: 19.04.2000
(51) Int. Cl.: C07H 15/04, C07H 15/08, A23L 1/236

(54) **CRYSTALLIZATION OF GLUCOPYRANOSYLALDITOLS, CRYSTALLINE GLUCOPYRANOSYLALDITOL PRODUCT AND USE THEREOF**
KRISTALLISATION VON GLUCOPYRANOSYLALDITOL, KRISTALLINES GLUCOPYRANOSYLALDITOL UND SEINE VERWENDUNG
CRISTALLISATION DE GLUCOPYRANOSYLALDITOLS, PRODUIT DE GLUCOPYRANOSYLALDITOL CRISTALLIN ET UTILISATION DERIVEE

(30) Priority: 23.04.1999 FI 990924
(43) Date of publication of application: 23.01.2002
(73) Proprietor: Danisco Sweeteners Oy, 02151 Espoo (FI)
(72) Inventor: HEIKKILÄ, Heikki, FIN-02320 Espoo (FI); NYGREN, Johanna, FIN-08700 Virkkala (FI); SARKKI, Marja-Leena, FIN-02460 Kantvik (FI); GROS, Hakan, FIN-02460 Kantvik (FI); EROMA, Olli-Pekka, FIN-48100 Kotka (FI); PEARSON, Julita, West Wickham, Kent BR4 0QA (GB); PEPPER, Tammy, Weybridge, Surrey KT13 9JZ (GB)
(74) Representative: Hjelt, Pia Dorrit Helene
(86) International application number: PCT/FI2000/000338
(87) International publication number: WO 2000/064916

(56) References cited:
- EP-A1- 0 854 148
- WO-A1-90/14821
- DE-A1- 19 705 664
- GB-A- 1 483 998
- US-A- 5 578 339

## Description

The present invention relates to a novel process for the crystallization of glucopyranosylalditols, to a particulate glucopyranosylalditol product having novel properties, to the use thereof in confectionery, foodstuffs and pharmaceuticals, and to special products comprising the same. The present invention specifically provides a product containing crystalline glucopyranosylalditol, wherein the crystals are produced by microcrystallization of glucopyranosylalditol from a liquid glucopyranosylalditol solution.

The glucopyranosylalditols of the present invention are selected from the group consisting of 1-O-α-D-glucopyranosyl-D-mannitol (1,1-GPM), 6-O-α-D-glucopyranosyl-D-sorbitol (1, 6-GPS) and 1-O-α-D-glucopyranosyl-D-sorbitol (1,1-GPS).

The glucopyranosylalditols are generally produced by hydrogenation of isomaltulose and trehalulose which in turn are produced by isomerization of sucrose by specific bacterial strains or enzymes. The most well known of the isomerizing bacterial strains are *Protaminobacter rubrum* (CBS 574,77) and *Erwinia rhapontici* (NCPPB 1578) which produce predominantly isomaltulose from sucrose, while some bacterial strains such as *Pseudomonas mesoacidophila* MX-45 (FERM BP 3619) and *Agrobacterium radiobacter* MX-232 (PERM BP 3620) are known to produce a larger proportion of trehalulose in the isomerization of sucrose as described, for instance, in US Patent 5,578,339 (Südzucker Aktiengesellschaft). The ratio between isomaltulose and trehalulose may also be adjusted by controlling the conversion conditions.

The isomaltulose and trehalulose may be hydrogenated as a mixture or they may be separated from each other prior to hydrogenation e.g. by crystallization, chromatography, etc. Hydrogenation of pure isomaltulose produces a mixture of 6-O-α-D-glucopyranosyl-D-sorbitol (1,6-GPS) and 1-O-α-D-glucopyranosyl-D-mannitol (1,1-GPM). Although the hydrogenation theoretically should yield an equimolar mixture of the two glucopyranosylalditols, it has been shown that the proportion of each isomer may vary between, for example, 43 % and 57 %. Hydrogenation of trehalulose produces a mixture of 1-O-α-D-glucopyranosyl-D-mannitol (1,1-GPM) and 1-O-α-D-glucopyranosyl-D-sorbitol (1,1-GPS).

The ratio of the different glucopyranosylalditols may be adjusted by adjusting the proportions of isomaltulose and trehalulose before hydrogenation e.g. by crystallization of isomaltulose from mixtures of the two, by selecting the bacterial strains as shown in US Patent 5,578,339 or by adjusting the hydrogenation conditions as shown in EP-A 1-0854148.

The glucopyranosylalditols may also be produced as individual compounds or they may be separated into such from mixtures thereof in a manner well known to those skilled in the art, e.g. by crystallization. Thus, any ratios of the three glucopyranosylalditols may be obtained as desired.

Both 1,6-GPS and 1,1-GPM have a sweet taste which has about half the sweetness of sucrose. Their substantially equimolar mixture is known as isomalt or Palatinit^{R} and has found use as a commercial sweetener. 1,1-GPS also has a sweet taste but in contrast to 1,1-GPM and 1, 6-GPS, 1,1-GPS has not been found to crystallize easily from water.

The glucopyranosylalditols are attractive as sugar substitutes in food contexts because of their metabolic, dental and technical characteristics. The glucopyranosylalditols have slightly less than half the sweetness of sugar and a caloric content which is less than that of sugar. By combining the glucopyranosylalditols with other sugar alcohols such as xylitol, sorbitol, maltitol or intense sweeteners a synergistic effect may be obtained which easily raises the sweetness of the product to that of sucrose as described in Carbohydrates in Industrial Synthesis, Edited by M.A. Clarke, Verlag Dr. Albert Bartens KG, Berlin, 1992, pages 37 to 55.

The glucopyranosylalditols, especially isomalt, have been used as sweetening agents resembling sugar. For instance, isomalt has been used in confectionery, bakery products, desserts, jams, chocolates, chewing gums, and in various dietetic products. The glucopyranosylalditols are metabolized largely independently of insulin, so they can be safely consumed by non-insulin dependent diabetics.

The production and properties of the glucopyranosylalditols has been described by M. Munir et al. in Carbohydrate Res. 164 (1987), pages 477-485. Reference is made also to US Patent 5,578,339. However, the present invention is not limited to any specific way of producing the glucopyranosylalditols individually or as a mixture.

Isomalt has been commercially produced in crystalline form by crystallization in a special equipment which provides a combination of vacuum evaporation, crystallization and drying (Carbohydrates in Industrial Synthesis, Edited by M.A. Clarke, Verlag Dr. Albert Bartens KG, Berlin, 1992, page 48). According to US Patent 5,578,339 (Südzucker Aktiengesellschaft) a liquid containing the three glucopyranosylalditols as well as mannitol and sorbitol may be obtained in a dry form by evaporation of the aqueous solvent. Increasing the proportion of 1,1-GPS in the glucopyranosylalditol mixture reduces the tendency of the glucopyranosylalditols to crystallize out in candies and jam.

1,6-GPS and 1,1-GPM have been separated from their mixtures by fractional crystallization, as described in GB Patent 1 483 998. Such a crystallization of the glucopyranosylalditols from a liquid such as from an aqueous solution requires specific crystallization conditions and fairly long crystallization times. Due to the nature of conventional crystallization methods all of the glucopyranosylalditols in the solution cannot be obtained in crystalline form. A part of the glucopyranosylalditols will always remain in the mother liquor and will be discarded with the mother liquor even after repeated series of crystallizations. This provides a comparatively low yield of the process. A possible presence of 1,1-GPS complicates the crystallization further.

Thus, there exists a need for improving the production of solid glucopyranosylalditol products and the present invention aims at satisfying that need.

The object of the present invention is thus to provide an improved process for the crystallization of glucopyranosylalditol(s).

An object of the present invention is also to provide a solid particulate glucopyranosylalditol product.

Another object of the present invention is to provide a particulate glucopyranosylalditol product in a process which transforms a glucopyranosylalditol solution into a solid glucopyranosylalditol product in one overall operation.

An object of the invention is also to provide a novel particulate glucopyranosylalditol product which is suitable for use in the food industry.

Consequently, the present invention, which is defined in the appended claims, provides a novel process for producing a solid particulate glucopyranosylalditol product. Said process comprises contacting a liquid containing one or more dissolved glucopyranosylalditols selected from the group consisting of 1-O-α-D-glucopyranosyl-D-mannitol (1,1-GPM) and 6-O-α-D-glucopyranosyl-D-sorbitol (1,6-GPS), and optionally 1-O-α-D-glucopyranosyl-D-sorbitol (1,1-GPS), with gas suspended fine solid particles containing one or more of said glucopyranosylalditols; causing substantial removal of the solvent component of said liquid and allowing the resulting glucopyranosylalditol material to form an essentially solid composition of matter comprising a multitude of microcrystals of said glucopyrano-sylalditol(s); and causing said glucopyranosylalditol composition to be conditioned during a drying step to provide a product with a multitude of microcrystal containing particles of glucopyranosylalditol(s) agglomerated together in a random manner.

In a preferred embodiment of the process the fine solid particles consist essentially of microcrystals of one or more of said glucopyranosylalditol(s), preferably microcrystals of 1,6-GPS and 1,1-GPM (isomalt). A specially preferred product of said process consists essentially throughout its entire structure of a multitude of microcrystals of one or more of said glucopyranosylalditol(s), especially of isomalt, agglomerated together in a random manner.

In a preferred embodiment of the invention an aqueous solution of glucopyranosyl-alditol(s) is brought into contact with fluidized particles containing microcrystalline glucopyranosylalditol(s), the wetted particles are dried in a flow of warm gas, and the glucopyranosylalditol(s) on the surface of the particles is/are allowed to form new microcrystals on said surface.

By further conditioning the particles, the microcrystallization is allowed to proceed for a sufficient time to provide a final product consisting substantially of microcrystalline glucopyranosylalditol(s). In cases where the glucopyranosylalditol is isomalt, or 1,1-GPM or 1,6-GPS alone, the resulting product preferably consists essentially throughout its entire structure of microcrystalline glucopyranosylalditol(s).

In a preferred embodiment of the invention the wetted particles are substantially dried while falling down with a co-current air stream and allowed to settle into a porous layer of agglomerated microcrystallizing glucopyranosylalditol(s), which is then conditioned and cooled. The microcrystallization conditions are selected so that the cooled layer is dry, porous and brittle. If desired, the layer may be broken up into smaller fractions. Only a mild crushing action is needed to break up the agglomerated mass of microcrystals. The agglomerated product will primarily be broken up at the interfaces between individual crystals rather than by disrupting the crystals themselves.

In another embodiment of the invention the particles are retained in a suspended state in a gas such as an air stream while additional solution is sprayed onto their surfaces until the particles have grown to a predetermined size or weight. The particles are then removed from the air stream, e.g. by gravity and conditioned as described above.

The gas suspended fine solid glucopyranosylalditol particles are preferably provided by recirculating a portion of the microcrystals containing product produced in the process itself. Said particles may comprise dust entrained in circulating drying air or it may be dust or fine particles provided by the crushing of the agglomerated microcrystal containing mass. At start-up, in the absence of microcrystal containing glucopyranosylalditol, the solid feed of the process may comprise milled crystalline glucopyranosylalditol from another source. This solid feed should, however, be progressively replaced by recycling part of the microcrystal containing product, in order to provide a total or in any case a substantial microcrystalline structure to the product.

The terms "microcrystalline" and "microcrystal" as used throughout the present specification and claims should be understood to mean very small crystals having a size which on an average is below 50µ, and generally is of the order of about 10µ, on an average. In contrast to the present microcrystals, the crystals obtainable by crystallization of 1,6-GPS or 1,1-GPM from liquid suspensions by prior known crystallization techniques are discrete crystals the particle size of which, on an average, is of the order of about 100-1000µ or larger.

Consequently, the present invention provides a novel particulate glucopyranosylalditol product wherein each product particle comprises a multitude of microcrystal containing particles of one or more glucopyranosylalditols selected from the group consisting of 1-O-α-D-glucopyranosyl-D-mannitol (1,1-GPM) and 6-O-α-D-glucopyranosyl-D-sorbitol (1,6-GPS), and optionally 1-O-α-D-glucopyranosyl-D-sorbitol (1,1-GPS) agglomerated together in a random manner.

In a preferred embodiment each particle throughout its entire structure consists essentially only of a multitude of microcrystals of glucopyranosylalditol(s).

Although the size of the particles according to the present invention is not critical and may vary according to the intended use of the product, the mean particle size of the glucopyranosylalditol product is generally between about 0.05 and 2.0 mm. The preferred mean particle size is generally about 0.15-0.4 mm. Each particle contains a mass of minute crystals. The particle size and distribution may be controlled to suit the intended use.

The glucopyranosylalditol product may be used as such as obtained directly from the process, it may be broken up or it may even be cast in the form of ordinary sugar lumps or cubes.

The individual 1,6-GPS microcrystals generally comprise anhydrous 6-O-α-D-glucopyranosyl-D-sorbitol, while the individual 1,1-GPM microcrystals generally comprise 1-O-α-D-glucopyranosyl-D-mannitol dihydrate. 1,1-GPS has not been found to crystallize easily from an aqueous solution and, consequently, the 1,1-GPS optionally included in the product will generally dry by evaporation of the solvent without crystallizing. Amorphous 1,1-GPS may thus be included in the mixture. The crystal mass may also include portions of amorphous 1,1-GPM and/or 1,6-GPS. In a preferred embodiment of the invention, the microcrystals consist essentially of an equimolar mixture of anhydrous 1,6-GPS and 1,1-GPM dihydrate, i.e. of crystalline isomalt.

It should be noted that the term "equimolar" mixture in this context is intended to cover also such mixtures where the glucopyranosylalditol isomers are present in a mixture of up to about 57 % of one and, correspondingly, about 43 % of the other, such a mixture being included in the term isomalt.

In case the liquid (and solid) feed contains only one or the other of 1,1-GPM and 1,6-GPS, then the resulting product particle will contain 1,1-GPM dihydrate or anhydrous 1,6-GPS, respectively.

The microcrystal containing glucopyranosylalditol product according to the present invention may be used as a bulk sweetener for the total or partial replacement of sucrose or other sweetening agents. Thus, it is useful in dietetic products, in confectionery, bakery products, cereals, desserts, ice cream, jams, beverages, etc., especially in chocolate, marzipan, hard candy and granulated or tabletted table top sweeteners.

The glucopyranosylalditol product according to the present invention is also useful in pharmaceuticals where it is preferably included as a sweetener, an excipient, a diluent and/or a carrier. The microcrystals containing product according to the present invention can be tabletted into a tablet.

A preferred embodiment of the present invention relates to a special sweetener which comprises microcrystals of glucopyranosylalditol(s). Such a sweetener may include other components such as excipients, binders and/or other sweeteners.

Such other sweeteners are preferably also non-cariogenic sweeteners such as intense sweeteners taken from the group comprising dipeptide sweeteners, saccharin, acesulfame K, aspartame, polyols, stevioside, cyclamate, sucralose and neohesperidin dihydrochalcone, etc. A combination of the present glucopyranosylalditol product with such sweeteners provides a synergistic sweetening effect which enhances the inherent sweetening power of the glucopyranosylalditol(s). Thus, for instance a combination of isomalt and about 10 to 25% of polyols such as xylitol, maltitol, lactitol and/or sorbitol provides a product having a sweetness similar to that of sucrose.

The preferred non-cariogenic sweetener according to the present invention consists essentially of microcrystalline isomalt or of microcrystalline isomalt in combination with xylitol, maltitol, lactitol and/or sorbitol.

Other components which may be used in the sweetener and/or other applications such as in pharmaceutical preparations may comprise, for instance, microcrystalline cellulose, carboxymethyl cellulose, polydextrose, dextrose, maltodextrin, lactose, sugar, etc. as well as other sugar alcohols.

The microcrystal containing glucopyranosylalditol product of the present invention is preferably produced in a pure isomalt form, i.e. containing throughout essentially microcrystalline isomalt.

The present microcrystal containing glucopyranosylalditol may, however, also be microcrystallized with other compounds. Thus, if the solid and/or liquid feed comprises other components, such as one or more of the above mentioned sweeteners or excipients, binders, active ingredients, etc. the product discharged from the microcrystallization apparatus will contain said other components). A secondary spray of another solid or liquid component may also be fed into the microcrystallization apparatus into contact with the microcrystallizing glucopyranosylalditol(s).

Only such additional components can be used which do not significantly and adversely interfere with the microcrystallization according to the present invention. Specifically, it is necessary that the additional components and the amounts thereof are selected so that the microcrystallizing particles will be substantially dry by the time they leave the suspended state. If the initially dried particles contain too much moisture, they will be clogged together forming large compact structures, wherein the microcrystallization throughout the product cannot be ensured.

Further embodiments of the present invention relate to products made from the novel microcrystal containing glucopyranosylalditol particles. Such products are typically edible products, pharmaceutical products and/or oral hygiene products such as those mentioned above. The microcrystal containing glucopyranosylalditol of the present invention may, for instance, be advantageously used in the production of hard candy and chewing gum or in technological use.

The present invention will now be described in greater detail. This description should, however, not be taken as limiting the invention to the precise wording thereof. A person skilled in the art will be able to provide numerous modifications and variations of the process without deviating from the invention as defined in the appended claims.

The microcrystal containing glucopyranosylalditol particles produced according to the present invention are shown in the accompanying drawing, wherein

Fig. 1 is a SEM photo showing the microcrystal containing glucopyranosylalditol structure in 200x magnification.

Fig. 2 is a SEM photo showing the microcrystal containing structure in 1000x magnification.

Fig. 3 is a SEM photo showing conventional crystalline 1,6-GPS in 200x magnification.

Fig. 4 is a SEM photo showing conventional crystalline 1,1-GPM in 200x magnification.

In the process according to the present invention a liquid containing dissolved glucopyranosylalditol(s) is provided. The solvent component of said liquid is preferably water, although the glucopyranosylalditol(s) may also be microcrystallized from other solvents such as alcohols, e.g. ethanol or isopropanol.

The glucopyranosylalditol concentration of said aqueous feed solution should be between about 30% by weight and about 80% by weight in order to quickly provide supersaturation for the crystallization. Said concentration is preferably about 50-70% by weight.

Prior to feeding the solution into a microcrystallization apparatus, the solution is preferably heated in order to facilitate the subsequent removal of the solvent component and in order to more quickly provide suitable crystallization conditions in said apparatus. An aqueous solution is preferably heated to a temperature of about 45-95 °C, preferably about 60-80°C prior to feeding into said apparatus.

Especially in cases where the solution has a fairly low concentration the temperature should be kept in the lower range in order to avoid dissolving the solid fine glucopyranosylalditol particles.

The solution should preferably be distributed in the form of small droplets in the microcrystallization apparatus. To this end, the solution is preferably fed at a pressure through a nozzle into said apparatus. In the apparatus the solution is brought into contact with solid particles containing microcrystalline glucopyranosylalditol which are simultaneously fed into the apparatus so as to be fluidized or suspended in air in said apparatus.

The fine solid glucopyranosylalditol particles used as dry feed should contain microcrystalline glucopyranosylalditol. Preferably the particles consist essentially of microcrystalline glucopyranosylalditol(s). These particles may be provided by recirculation from the microcrystallization apparatus. Most preferably a fine fraction of the product is recirculated. Such a fine fraction typically has a mean particle size below about 0.2 mm, preferably below about 0.1 mm. However, when larger individual product particles are desired, correspondingly larger glucopyranosylalditol particles may be recirculated or fed into the apparatus from another source. The dry solid particles may also be dust or fine particles entrained in the drying air and fed back into the apparatus as solid feed.

The solution is generally contacted with the gas suspended solid particles in an upper portion of the microcrystallization apparatus. Here the wetted particles and any free droplets of glucopyranosylalditol solution meet a drying gas such as heated air which is introduced into the apparatus to provide removal of the solvent component of said solution. The drying air is preferably heated to a temperature of about 45-250°C, preferably about 70-160°C.

The drying should be accomplished in such a way as to substantially remove the solvent while said glucopyranosylalditol material is still in a gas suspended state. When the solvent is water, said drying should provide a suspended glucopyranosylalditol material dried to a free moisture content of about 0.1 to 2%, preferably below 1%. The free moisture is calculated as any water which is not bound as crystal water in the microcrystallizing glucopyranosylalditol.

In case the drying is not sufficient or too much liquid has been fed into the apparatus, the glucopyranosylalditol material will be too wet and the crystals will stick together to form a dense or syrup-like structure where separate microcrystals can no longer be properly discerned.

A substantial amount of solid glucopyranosylalditol feed is required in order to obtain a satisfactory particulate microcrystal containing product. The suitable ratio of liquid glucopyranosylalditol feed to solid glucopyranosylalditol feed varies with the microcrystallization conditions. The ratio should be selected so as to provide a wetting of the solid particle surfaces without dissolving the core of the seed particles. The amount of liquid component also depends on the ease of volatilization of the solvent and on the temperature of the liquid feed as well as the temperature and amount of the drying gas.

The suspended wetted particles may be dried by a co-current or a counter-current stream of drying air. The co-current air will flow downwards with the falling particles while a counter-current air stream will retain the particles in a gas suspended or fluidized state for a longer time.

The particles carried downwards with a co-current air stream in a microcrystallization apparatus should be substantially dry by the time they reach the bottom portion of the apparatus and are allowed to settle there. The settling surface is preferably a means allowing building up of a suitable layer and for adjusting the reaction time in the layer. A belt moving at a speed sufficient to allow build up of a porous agglomerated layer of glucopyranosylalditol(s) is generally suitable. The layer typically has a thickness of about 0.5 to 15 cm, preferably about 3-7 cm. The layer should preferably be so porous that air easily penetrates therethrough.

The agglomerated layer of solidified glucopyranosylalditol should further be conditioned so as to allow microcrystallization to proceed in the layer. Said conditioning preferably includes two or more separate steps or phases with different temperatures. The layer is preferably treated e.g. by blowing a hot drying gas therethrough. The temperature and amount of the drying gas is selected so as to provide suitable microcrystallization conditions in the layer. The temperature of the drying gas is typically about 40-150°C, preferably about 50-90°C.

The conditioning may preferably be performed in several successive steps with, for instance, decreasing temperatures of the drying gas so as to ensure a proper drying and microcrystallization of the glucopyranosylalditol.

The conditioning should continue for a sufficient time to allow microcrystallization of any liquid glucopyranosylalditol to take place in the layer. Typically, the conditioning should continue for a time of about 10-180 min or more, preferably about 20-40 min. After conditioning a stable product is obtained.

After conditioning, the agglomerated particle layer is preferably post-conditioned while being allowed to dry completely. When microcrystallized glucopyranosylalditol is recirculated within the process, care should be taken to recirculate only essentially dry particles. Problems may occur if the dry feed comprises recycled material which is moist. The microcrystallized layer should thus be properly dried before comminuting.

If the surface on which the layer is allowed to settle is flat, the result will be a substantially flat porous and brittle plate comprising microcrystalline glucopyranosylalditol(s). However, the microcrystallizing glucopyranosylalditol product may also be gathered in moulds having any desired form such as resembling ordinary sugar lumps, or bars, strings, cubes, spades, hearts, flowers, etc.

When the microcrystal containing product is in the form of a continuous layer, it is generally desirable to break up the agglomerated layer to provide discrete particles. Only a mild comminuting action is required for breaking up the bonds between individual microcrystals. The glucopyranosylalditol product is preferably comminuted while warm.

The resulting microcrystal containing glucopyranosylalditol particles are preferably fractionated after an eventual milling and a portion thereof is recirculated to provide a feed of solid particles containing microcrystalline glucopyranosylalditol into the microcrystallization apparatus.

Generally the microcrystal containing glucopyranosylalditol particles are broken up so as to provide particles having a mean particle size of about 0.05 to 2.0 mm, preferably about 0.15-0.4 mm. It is generally desirable to recirculate fine particles having a mean particle size below about 0.2 mm, preferably below about 0.1 mm, although larger particles may be recirculated, especially in cases where the desired end product comprises larger particles.

In the case where the drying air is blown counter-currently to the downward movement of the wetted particles in the microcrystallization apparatus, the particles will be fluidized therein. By a suitable fluidization action the particles will be made to recirculate within the apparatus. In the apparatus a simultaneous wetting, drying and microcrystallization of particles will take place. Each particle will pass through several wetting and drying/micro-crystallization stages, colliding with other particles and growing ever bigger until the particle reaches the size and weight wherein the fluidizing air no longer manages to retain them in a fluidized state. At this stage the particles will fall to the bottom of the apparatus and may be removed therefrom to be conditioned, for instance as described above.

The solid feed to the microcrystallization apparatus in the counter-current case preferably comprises dust and fine particles recovered from the circulation of drying air.

In the particulate microcrystal containing glucopyranosylalditol product according to the present invention each particle substantially throughout its entire structure comprises a multitude of microcrystals of glucopyranosylalditol agglomerated together in a random manner. The total glucopyranosylalditol purity of the product is preferably more than 80%, preferably more than 90%, most preferably up to 98% or more.

In the preferred process about 10-90%, preferably about 30-70%, most preferably about 40 to 60% of the dry substance derives from a feed of solid microcrystal containing particles, preferably recirculated from the production line or from the drying air. A substantial amount of solid feed is essential in glucopyranosylalditol microcrystallization. The co-current system often requires slightly more solid feed than the counter-current system. Thus, for the co-current drying system, the preferred amount of dry substance deriving from the solid particles is 50-80%.

The microcrystals in each product particle of the present invention are individually very small compared to the crystals formed by prior art crystallization processes. Generally, the size of the microcrystals in each particle is on an average below 50µ, preferably about 10µ on an average.

Although 1,6-GPS crystallizes predominantly in the anhydrous crystal form and 1,1-GPM as a dihydrate in the process according to the present invention, also some amorphous glucopyranosylalditol(s) may be present. The degree of crystallinity of a microcrystalline isomalt product is, however, generally high, generally as high as 90 % or more.

The water content of the preferred microcrystalline isomalt product varies according to production parameters in the range of about 3 to 7 %. The product comprises dihydrate and anhydrous crystals although some of the dihydrate crystals may be partly or totally dehydrated.

The invention will now be illustrated with the aid of a few examples. These examples should in no way be taken as limiting the invention.

### Example 1

### Microcrystallization of isomalt

An isomalt solution (dry solid concentration 54.1 % by weight, purity over 98 % on dry substance with a 1,1-GPM to 1,6-GPS ratio of approximately 1:1) was fed into a heated feed tank. The temperature of the solution in the feed tank was kept at 65 to 68°C. Solution was supplied from the tank to a spray nozzle at a rate of 36.9 kg/h. The feed pressure of the solution varied from 100 to 140 bar.

Simultaneously with the solution small particles of dry crystalline isomalt were fed to the microcrystallization apparatus at a rate of 19.3 kg/h. Drying air was also fed into the apparatus to dry the sprayed solution and wetted particles. The temperature of the air was adjusted to about 90 to 112 °C. The partly dried mixture of liquid and dry feed fell co-currently with the air-stream towards a screen having a temperature of 55 to 72 °C.

The apparatus was operated under these conditions for 9 minutes. During this time an agglomerated, porous fluffy layer having a thickness of about 5 cm built up on the screen. The isomalt layer was conditioned on the screen for about 30 minutes and the temperature dropped slowly from 67 °C to 45 °C. The microcrystallized product was collected from the screen, subjected to a gentle milling at about 40°C and sieved.

The water content of the microcrystalline isomalt product was found to be about 6.2 % by the Karl Fischer method.

### Example 2

### Microcrystallization of isomalt

The procedure of Example 1 was repeated several times under varying test conditions. The solid feed comprised recirculated microcrystalline isomalt from Example 1. The test conditions are indicated in Table 1.

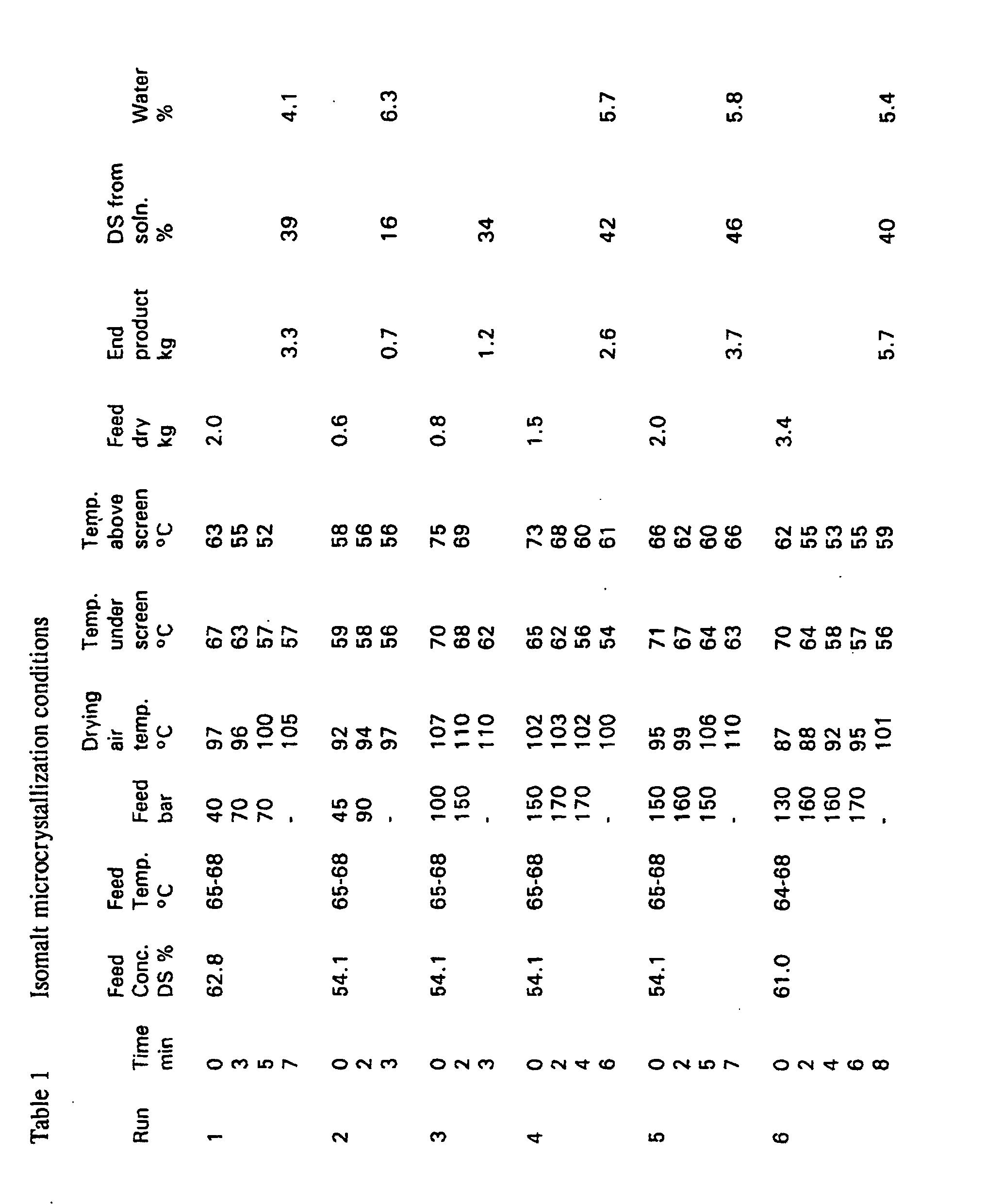

Dry feed is essential in isomalt microcrystallization. Problems occurred when the dry feed was a recycled material which was moist. Cooling when moist made the layer hard. The microcrystallized layer was warm at milling.

### Example 3

### Microcrystallization of 1,6-GPS

A continuous fluid bed 1,6-GPS microcrystallization is performed in an apparatus having a fluid bed drying chamber, equipped with a spray nozzle system inside in the middle of the chamber. The apparatus comprises a bottom screen with a hole for the discharge of the heaviest particles, and a cyclone to recover light particles.

The chamber is loaded with 1 kg of powdered 1,6-GPS to act as seed material for the microcrystallization of 1,6-GPS. The powdered 1,6-GPS is fluidized with a flow of air (temperature 55-85 °C) through the bottom screen. A 1,6-GPS solution (concentration 60 %, purity over 99 % D.S.) at a temperature of 70 °C is fed into the chamber with a pump, atomized by means of a nozzle and sprayed over the fluidized isomalt powder.

The solution is supplied at a rate of 1 kg/h at a pressure of 1.5 bar to the fluidized 1,6-GPS powder. The air flow rate is adjusted to fluidize the 1,6-GPS and to evaporate water at a rate sufficient to crystallize the 1,6-GPS. A microcrystalline agglomerate is formed when the 1,6-GPS crystallizes around the powder particles. The agglomerates remain in a fluidized state until they fall down when their weight is high enough. 1,6-GPS agglomerates are discharged continuously through the bottom opening.

In the drying chamber the lightest, non-agglomerated 1,6-GPS particles are removed from the top of the chamber entrained in the exiting air stream. This fine 1,6-GPS material is recovered in a cyclone and fed back to the chamber to act as a continuous seed stream.

The discharged agglomerated product is conditioned at a temperature of 45-50°C for 30 minutes to balance the microcrystallization.

Steady state conditions are reached when all the powdered 1,6-GPS used as a starting seed has been discharged from the process. The product obtained thereafter is a totally microcrystalline product which throughout its entire structure consists of microcrystalline 1,6-GPS.

### Example 4

### Chewing gum

A microcrystalline isomalt product produced according to the process of Example 1 having a mean particle size of about 200 µm was evaluated in a standard stick chewing gum production and compared to a chewing gum made from commercially available isomalt having a particle size wherein 90% was < 100 µm.

The following ingredients were used:

| Ingredient | % Fresh Basis |
|---|---|
| Gum base, Nova Base (Dreyfus) | 25.0 |
| Sorbitol Syrup, Sorbifin LS (Xyrofin) | 7.2 |
| a) Isomalt PF (Südzucker AG) | 47.0 |
| b) Microcrystalline isomalt | 47.0 |
| Mannitol, milled (Cerestar) | 8.0 |
| Glycerine (Henkel) | 10.0 |
| Peppermint Flavour liquid (IFF) | 1.2 |
| Peppermint Flavour powder (IFF) | 1.6 |
| Aspartame (Nutrasweet) | q.s (0.05) |

The isomalt a) was commercial crystalline isomalt and isomalt b) was microcrystalline isomalt produced according to the present invention.

The chewing gums were produced by placing the isomalt powder in a Z-blade mixer and mixing in the softened gum base at a mixer temperature of 40 °C. The ingredients were thoroughly combined before the next ingredient was added. Then the sorbitol liquid was added followed by the mannitol and the powdered flavour.

The glycerin was combined with the liquid flavour and aspartame and added to the mixer. As soon as a homogeneous paste was formed, it was removed from the mixer and dusted with milled mannitol. The paste was laminated to required thickness and cut.

The commercial crystalline isomalt gum processed well in about 12 minutes. The finished gum had a good chew and posed no problems during processing. The microcrystalline isomalt gum processed slightly quicker, in about 10 minutes. The paste produced was very soft and the finished gum had a softer texture than the batch produced with the commercial crystalline isomalt. This can be attributed to the larger particle size of the material. No grittiness was detected when chewing the sample.

As can be seen from the above results the microcrystalline isomalt is suitable for use in a stick chewing gum.

### Example 5

### Hard candy

Microcrystalline isomalt product produced according to the process of Example 1 was evaluated in a standard hard candy production and compared to a hard candy made from a commercially available isomalt (Isomalt Type PF, Südzucker AG).

A 1.5 kg batch (dry substance) of 1) 100 % Isomalt Type PF and 2) microcrystalline isomalt was diluted to 85 % solids with water and placed in a sauce pan. The batch was first heated on a hot plate until all the material was solubilized. The batch was then transferred to a vacuum cooker and heated to approximately 160 to 165 °C before a vacuum of 0.3 bar was applied for 10 minutes. The syrup was then transferred from the cooker to a slab and tempered until a suitable texture for drop rolling was achieved. The tempered mass was fed through a drop roller and the finished candies were wrapped in cellophane after a few minutes cooling time and some of the candies from each batch was left unwrapped. The syrup viscosity, the tempering time, the handling characteristics of the mass, the temperature of the mass prior to drop rolling and the finished candy quality were noted.

Moisture contents of the finished samples were measured using a Mettler Toledo DL35 Karl Fischer titrator.

Accelerated storage trials were carried out at 55 % relative humidity at 20 °C and at 70 % relative humidity at 20 °C on unwrapped samples. The samples were weighed periodically and an average % weight gain for each product was calculated. At the end of the test period the overall quality of the candies was assessed. The results are indicated in Table 2.

**Table 2.**

| Analysis results | | | | |
|---|---|---|---|---|
| Batch | Boiling time | Tempering time | Forming temp. | % Solids |
| Isomalt PF | 16 min 35 s | 6 min 40 s | 76 °C | 99.1 % |
| | | | | |
| Microcryst. isomalt | 14 min 57 s | 6 min 10 s | 82 °C | 99.3 % |

Both batches handled very similarly in the drop roller. In the storage trials at 55 % RH at 20 °C both samples appeared similar after 76 days. Both were clear and showed no changes from their original appearance. The weight gains of the samples were virtually the same. However, the samples stored at 75 % RH showed some differences. Both samples had become dull and cloudy with a waxy layer on the outer surfaces with the samples of Isomalt PF having some white patches around the edges. The Isomalt PF samples had gained 2.9 % whereas the microcrystalline isomalt had gained only 2.2 % weight.

The overall impression was that microcrystalline isomalt performed very similarly to Isomalt Type PF.

### Example 6

### Chocolate

A mixture of microcrystalline 1,6-GPS and 1,1-GPS (isomalt) produced according to the procedure described in Example 1 was assessed in a standard chocolate production and compared to chocolate made from commercially available milled grade isomalt.

The following ingredients were used

| | |
|---|---|
| a) Microcrystalline isomalt | 46.0% |
| b) Isomalt PF (Südzucker AG) | 46.0% |
| Cocoa Liquor (BCM) | 42.0% |
| Cocoa Butter (BCM) | 11.8% |
| Lecithin (Lucas Myer) | 0.2% |

The sweeteners a) and b) were each mixed with the cocoa liquor. The resulting mixture was then passed through a three roll refiner to produce a flake. The flake was mixed again and a part of the cocoa butter was added. The mix was re-refined with increased pressures to produce a flake with a fat content of 32 %. The resulting flakes were stored prior to conching.

Both batches of flake were heated to 40°C. This temperature heated the flake sufficiently for ease of processing without over-heating. The batches were loaded into a conch and the remaining cocoa butter was added to produce a final chocolate with 35 % fat. All lecithin was added one hour prior to removal of the batch from the conch. Both batches had similar performance.

### Example 7

### Physical properties of isomalt

A batch of microcrystalline isomalt produced in accordance with the procedure described in Example 1 was analyzed as to its physical properties and compared with those of commercial grade isomalt (Isomalt F, Südzucker AG).

The following analysis methods were used:
**Moisture** was measured using coulometric Karl Fischer titration
**DSC** analysis was made at a speed of 10°C/minute
**Flowability:** A 500 g sample was poured into a a dry funnel whose bottom opening was blocked. The bottom opening was unblocked and the time needed for the entire sample to flow out of the funnel was measured.
**Bulk density:** The sample was poured to 500 ml measuring cylinder. The sample was tapped 10 times, levelled and the amount of the sample was weighed.
**Hygroscopicity:** 10 g of the sample was weighed to a petri dish. The open dish was put into a humidity cabinet. The change in weight was measured. Humidity cabinets at 25 °C and relative humidity 60 % and at 40 °C and relative humidity 70 % were used.
**Particle size distribution:** Sieve analysis was used to determine the particle size.
**Dissolving rate:** 100 g of the sample was put to 100 g of water at 20 °C and 40 °C. A small paddle mixer, 250 rpm, was used to mix the solution. During the dissolution the refractive index was measured, and the time for dissolving was recorded.
**Heat of solution:** 40 g of the sample was dissolved in 670 g of distilled water at 25°C. The heat of solution was measured with a calorimeter operating in a constant temperature environment.

The analysis results are shown in Table 3.

**Table 3.**

| Isomalt: Analysis results | | |
|---|---|---|
| **Analysis** | **Microcryst. isomalt** | **Isomalt F** |
| Moisture, % | 5.5 | 2.5 |
| DSC, 10 °C/min, peak at °C | 102.0 | 94.2 |
| | 154.2 | 147.3 |
| | | |
| Flowability, s | 18 | 15 |
| | | |
| Bulk density, g/500 ml | 340 | 418 |
| | | |
| Heat of solution, cal/g | 12.8 | 7.0 |
| | | |
| Hygroscopicity | | |
| at 25°C, % H₂O/48h | 0.22 | 1.19 |
| RH 60%, % H₂O/216h | 0.23 | 2.05 |
| at 40°C, % H₂O/48h | 0.69 | 3.07 |
| RH 70%, % H₂O/216h | 0.75 | 3.15 |
| Sieve analysis | | |
| > 0.710 mm | 17.0 | 2.3 |
| > 0.560 mm | 21.6 | 25.8 |
| > 0.450 mm | 14.9 | 31.7 |
| > 0.315 mm | 14.2 | 30.0 |
| > 0.250 mm | 13.6 | 7.3 |
| > 0.180 mm | 12.2 | 2.9 |
| > 0. 100 mm | 6.0 | 0.2 |
| 0 | 0.6 | 0.1 |
| | | |
| Mean particle size, mm | 0.48 | 0.48 |
| Coefficient of variation, % | 39 | 26 |

The total crystallinity of the microcrystalline isomalt determined from DSC-grams was over 65%. The difference in hygroscopicity between the microcrystalline isomalt and the commercial product was significant.

### Example 8

### Crystalline structure of the microcrystalline glucopyranosylalditols

SEM photos were taken of the microcrystalline isomalt produced according to Example 1 as well as of crystalline 1,6-GPS and 1,1-GPM crystallized from an aqueous solution in a conventional manner.

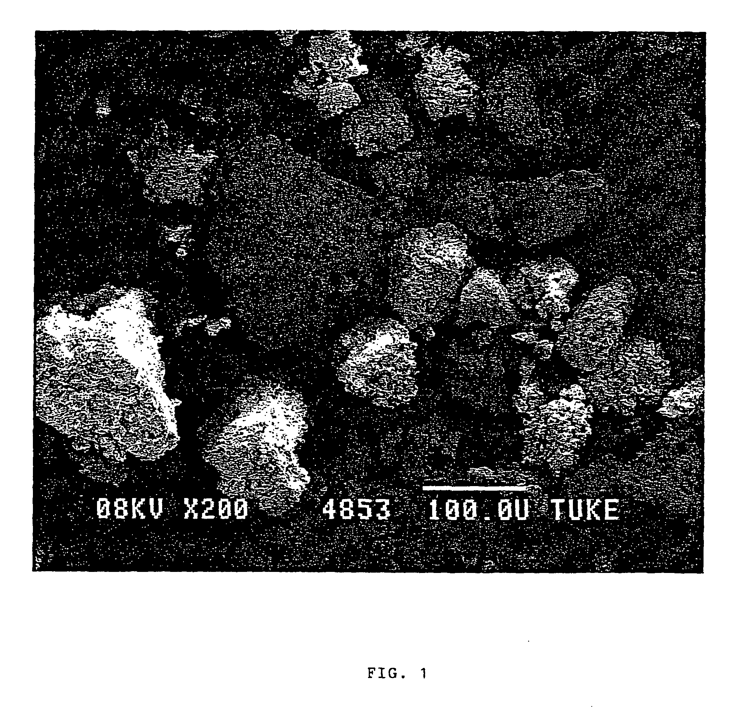

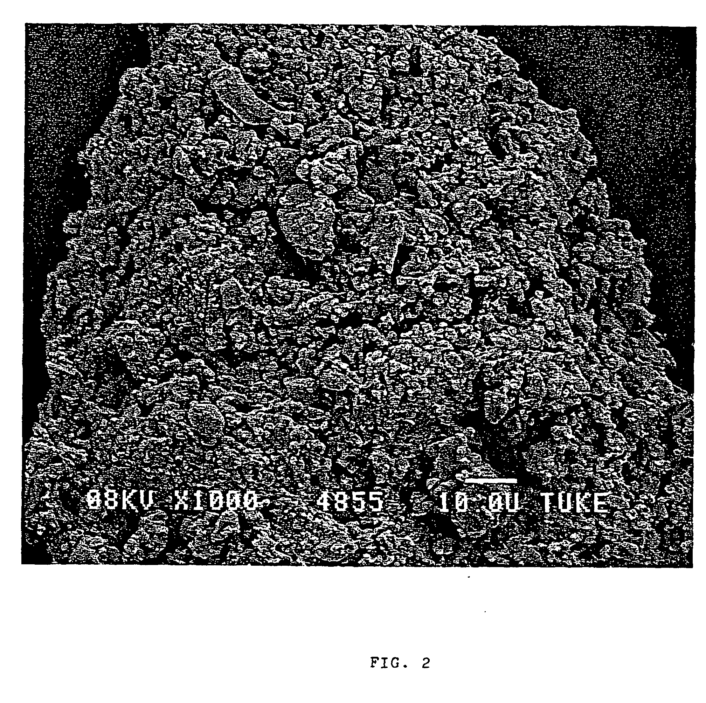

The microcrystalline isomalt in SEM photos in 200x magnification (Fig 1) showed a myriad of small crystals looking like normal crystal lumps but smaller. The microcrystalline structure showed very clearly in 1000x magnification (Fig. 2).

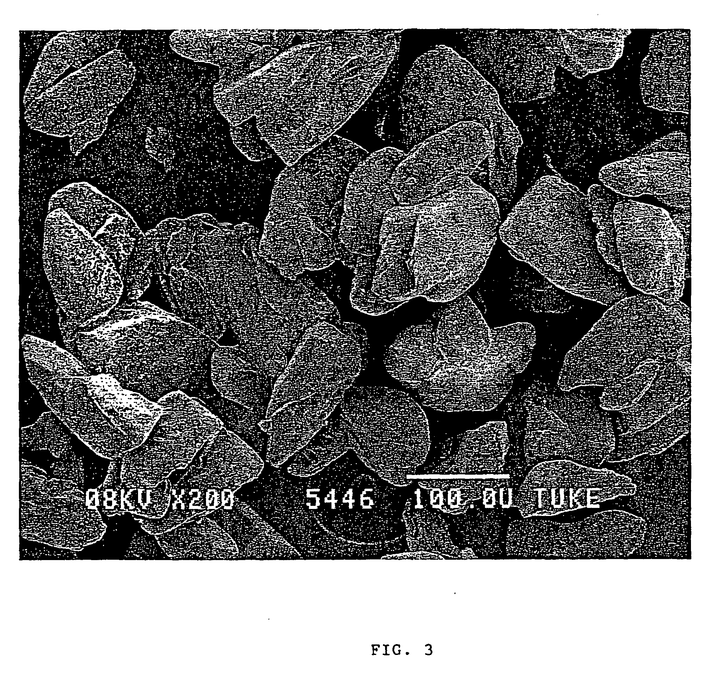

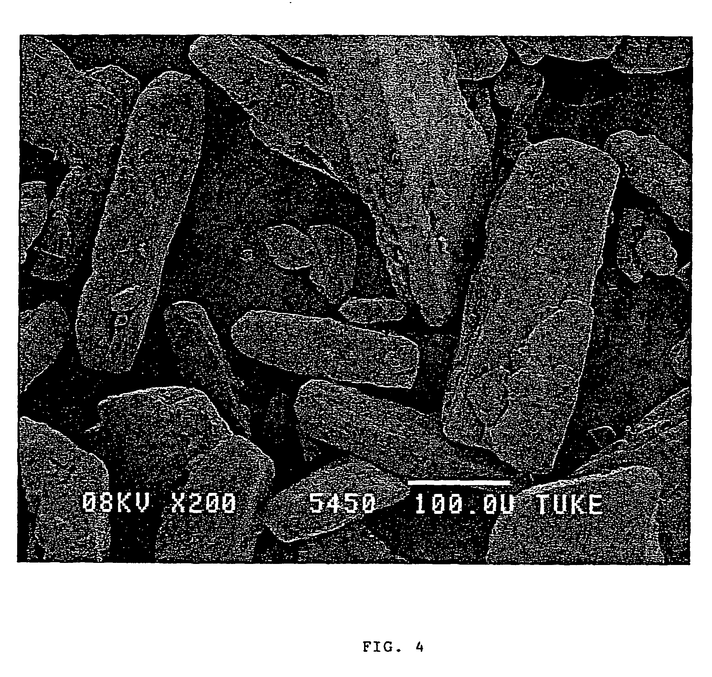

Comparable SEM photos of conventional crystalline 1,6-GPS and 1,1-GPM in 200x magnification (Figs 3 and 4, respectively) indicate the difference in the crystal size.

## Claims

1. A process for the crystallization of glucopyranosylalditols, comprising
- contacting a liquid containing one or more dissolved glucopyranosylalditols selected from the group consisting of 1-O-α-D-glucopyranosyl-D-mannitol (1,1-GPM) and 6-O-α-D-gluco-pyranosyl-D-sorbitol (1,6-GPS) and optionally 1-O-α-D-glucopyranosyl-D-sorbitol (1,1-GPS), with gas suspended fine solid particles containing one or more of said glucopyranosylalditols for wetting the particles;
- causing substantial removal of the solvent component of said liquid and allowing the resulting glucopyranosylalditol material to form an essentially solid composition of matter comprising a multitude of microcrystals of said glucopyranosylalditol(s); and
- causing said glucopyranosylalditol composition to be conditioned during a further drying step to provide a product with a multitude of microcrystal containing particles of gluco-pyranosylalditol(s) agglomerated together in a random manner, said microcrystals having on an average a size below 50µ.

2. The process according to claim 1, wherein said gas suspended fine solid particles consist essentially of microcrystals of one or more of said glucopyranosylalditol(s).

3. The process according to claim 2, wherein said product consists essentially throughout its entire structure of a multitude of microcrystals of one or more of said glucopyranosylalditol(s) agglomerated together in a random manner.

4. The process according to any one of claims 1, 2 or 3, wherein said glucopyranosylalditol is 1-O-α-D-glucopyranosyl-D-mannitol (1,1-GPM) or 6-O-α-D-glucopyranosyl-D-sorbitol (1,6-GPS) or a mixture thereof, preferably an equimolar mixture thereof (isomalt).

5. The process according to any one of the preceding claims, wherein said liquid is an aqueous solution of one or more glucopyranosylalditols having a glucopyranosylalditol concentration of about 30-80% by weight, preferably about 50-70% by weight.

6. The process according to any one of the preceding claims, comprising heating said liquid to a temperature of about 45-95 °C, preferably about 60-80 °C prior to said contacting.

7. The process according to any one of the preceding claims, wherein said contacting comprises spraying said liquid into contact with said gas suspended fine solid particles.

8. The process according to any one of the preceding claims, wherein said liquid contains a minor portion of an excipient, a binder, an active ingredient and/or other sweetener than a glucopyranosylalditol.

9. The process according to any one of the preceding claims, wherein a secondary spray of another liquid containing an excipient, a binder, an active ingredient and/or other sweetener is simultaneously provided.

10. The process according to any one of the preceding claims, wherein said removal of said solvent is performed by the introduction of a drying gas such as air heated to a temperature of about 45-250 °C, preferably about 70-160 °C.

11. The process according to claim 10, wherein said solvent is water and said solvent removal provides a glucopyranosylalditol material dried to a free moisture content of about 0.1 to 2 %, preferably below 1% while said glucopyranosylalditol material is still in a gas suspended state.

12. The process according to any one of the preceding claims, wherein said conditioning is maintained so as to allow glucopyranosylalditol microcrystallization to proceed in said composition.

13. The process according to any one of the preceding claims, wherein said glucopyranosylalditol composition is allowed to settle on a moving belt and to form thereon a substantially continuous agglomerated porous layer having a thickness of about 0.5-15 cm, preferably about 3-7 cm.

14. The process according to claim 13, wherein said conditioning includes treating said composition in said agglomerated layer with a drying gas having a temperature of about 40-100°C, for a time of about 10-180 min or more.

15. The process according to claim 14, wherein said conditioning is performed in several successive steps with decreasing drying gas temperatures. .

16. The process according to claim 14 or 15, which further comprises cooling said conditioned agglomerated layer to provide a flat porous and brittle plate comprising microcrystalline glucopyranosylalditol(s).

17. The process according to claim 15 or 16, comprising subjecting said plate to a comminuting action so as to break up said agglomerated layer, and optionally comminuting the product.

18. The process according to any one of the preceding claims, which further comprises fractionating said microcrystalline particles and recirculating at least a portion thereof to provide a feed of said fine solid particles containing microcrystalline glucopyranosyl-alditol(s).

19. The process according to any one of the preceding claims, wherein about 30-70%, preferably about 40-60% of the dry substance derives from a feed of solid microcrystalline particles.

20. The process according to any one of claims 1 to 11, wherein said solid particles are retained in a fluidized state until they have grown to a predetermined weight.

21. The process according to any one of the preceding claims, comprising recirculating microcrystalline glucopyranosylalditol particles having a mean particle size below about 0.2 mm, preferably below about 0.1 mm.

22. The process according to any one of the preceding claims, wherein said microcrystalline glucopyranosylalditol is microcrystalline dihydrate of 1-O-α-D-glucopyranosyl-D-mannitol (1,1-GPM) or anhydrous 6-O-α-D-glucopyranosyl-D-sorbitol (1,6-GPS) or a mixture thereof, preferably a substantially equimolar mixture thereof (isomalt).

23. A particulate glucopyranosylalditol product wherein each product particle comprises a multitude of microcrystal containing particles of one or more glucopyranosylalditols selected from the group consisting of 1-O-α-D-glucopyranosyl-D-mannitol (1,1-GPM) and 6-O-α-D-glucopyranosyl-D-sorbitol (1,6-GPS) and optionally 1-O-α-D-glucopyranosyl-D-sorbitol (1,1-GPS) agglomerated together in a random manner, said microcrystals having on an average a size below 50µ.

24. The glucopyranosylalditol product according to claim 23, wherein said product particle consists essentially throughout its entire structure of a multitude of microcrystals of one or more of said glucopyranosylalditol(s) agglomerated together in a random manner.

25. The glucopyranosylalditol product according to claim 23 or 24, wherein said micro-crytals consist of 1-O-α-D-glucopyranosyl-D-mannitol dihydrate or anhydrous 6-O-α-D-glucopyranosyl-D-sorbitol, or a mixture thereof, preferably an equimolar mixture thereof (isomalt).

26. The glucopyranosylalditol product according claim 23 or 24, wherein said microcrystals consist essentially of anhydrous 6-O-α-D-glucopyranosyl-D-sorbitol.

27. The glucopyranosylalditol product according to claim 23 or 24, wherein said microcrystals consist essentially of 1-O-α-D-glucopyranosyl-D-mannitol in the form of the dihydrate or in a partly or totally dehydrated form.

28. The glucopyranosylalditol product according claim 23 or 24, wherein said product particle contains amorphous 1-O-α-D-glucopyranosyl-D-sorbitol.

29. The glucopyranosylalditol product according to claim 23, having a total 1-O-α-D-glucopyranosyl-D-mannitol, 6-O-α-D-glucopyranosyl-D-sorbitol and 1-O-α-D-glucopyranosyl-D-sorbitol content of more than 80%, preferably more than 90%, most preferably 98% or more.

30. The glucopyranosylalditol product according to claim 24, said product particles having been produced by microcrystallization of a liquid containing dissolved glucopyranosylalditol(s) together with fine gas suspended solid particles containing microcrystalline glucopyranosylalditol(s).

31. The glucopyranosylalditol product according to claim 23, wherein about 10-90%, preferably about 30-80%, most preferably 40-60% of the dry substance of the final product derives from a feed of solid microcrystalline particles.

32. The glucopyranosylalditol product according to any one of the preceding claims 23 to 31, comprising product particles having a mean particle size of about 0.1-2 mm, preferably about 0.15-0.4 mm.

33. The glucopyranosylalditol product according to any one of the preceding claims 23 to 32, wherein the size of the microcrystals in each particle is on an average below 50µ, preferably about 10µ.

34. The glucopyranosylalditol product according to any one of the preceding claims 23 to 33, wherein said product particle additionally contains integrally in its structure additional components such as excipients, binders, active ingredients and/or other sweeteners.

35. Use of the microcrystal containing glucopyranosylalditol product according to claim 23 as a bulk sweetener for the total or partial replacement of sucrose.

36. Use of the microcrystal containing glucopyranosylalditol product according to claim 23 in confectionery, bakery products, cereals, desserts, jams, beverages, chocolate, marzipan, table top sweeteners, chewing gum, ice cream, and dietetic products as well as in pharmaceutical products.

37. The use according to claim 36 wherein said glucopyranosylalditol product is used in hard candies. .

38. Use of the microcrystal containing glucopyranosylalditol product according to claim 23 in a non-cariogenic chewing gum.

39. A special sweetener which comprises microcrystal containing glucopyranosylalditol according to claim 23.

40. The special sweetener according to claim 39 which is mainly composed of microcrystalline dihydrate of 1-O-α-D-glucopyranosyl-D-mannitol (1,1-GPM) or anhydrous 6-O-α-D-glucopyranosyl-D-sorbitol (1,6-GPS) or a mixture thereof, preferably a substantially equimolar mixture thereof (isomalt).

41. The special sweetener according to claim 39 which additionally contains other sweeteners selected from the group consisting of xylitol, maltitol, lactitol and sorbitol.

42. A glucopyranosylalditol chewing gum **characterized in that** it contains one or more glucopyranosylalditol(s) selected from the group consisting of microcrystalline 1-O-α-D-glucopyranosyl-D-mannitol dihydrate, microcrystalline anhydrous 6-O-α-D-glucopyranosyl-D-sorbitol and 1-O-α-D-glucopyranosyl-D-sorbitol, said microcrystals having on an average a size below 50µ.

43. The chewing gum according to claim 42, wherein said microcrystalline glucopyranosylalditol has been produced by contacting suspended microcrystalline glucopyranosylalditol particles with a glucopyranosylalditol solution, drying the resulting composition to cause glucopyranosylalditol microcrystallization, and conditioning said composition to provide a product consisting essentially throughout its entire structure of a multitude of microcrystals of glucopyranosylalditol agglomerated together in a random manner.

44. A glucopyranosylalditol hard candy, **characterized in that** it is made from a glucopyranosylalditol product obtainable according to the process of claim 1 and produced by contacting gas suspended particles comprising one or more microcrystalline glucopyranosylalditol(s) selected from the group consisting of 1-O-α-D-glucopyranosyl-D-mannitol dihydrate and anhydrous 6-O-α-D-glucopyranosyl-D-sorbitol with a solution of one or more glucopyranosylalditol(s) selected from the group consisting of 1-O-α-D-glucopyranosyl-D-mannitol, 6-O-α-D-glucopyranosyl-D-sorbitol and/or 1-O-α-D-glucopyranosyl-D-sorbitol, drying the resulting composition to cause glucopyranosylalditol microcrystallization, and conditioning said composition to provide a product with a multitude of microcrystal containing particles of glucopyranosylalditol(s) agglomerated together in a random manner.

## Patentansprüche

1. Verfahren zur Kristallisation von Glucopyranosylalditolen, das folgendes umfasst:
• Kontaktieren einer Flüssigkeit, die ein oder mehrere aufgelöste Glucopyranosylalditole, ausgewählt aus 1-O-α-D-Glucopyranosyl-D-mannit (1,1-GPM) und 6-O-α-D-Glucopyranosyl-D-sorbit (1,6-GPS) und gegebenenfalls 1-O-α-D-Glucopyranosyl-D-sorbit (1,1-GPS), enthält, mit gassuspendierten feinen Feststoffteilchen, die ein oder mehrere der Glucopyranosylalditole enthalten, zur Benetzung der Teilchen;
• Bewirkung der weitgehenden Entfernung der Lösungsmittelkomponente aus der Flüssigkeit und Ausbildung einer im wesentlichen festen Materialzusammensetzung aus dem resultierenden Glucopyranosylalditol-Material, das eine Vielzahl von Mikrokristallen aus dem/den Glucopyranosylalditol(en) umfasst; und
• Konditionierung der Glucopyranosylalditol-Zusammensetzung während eines weiteren Trocknungsschrittes, wodurch ein Produkt mit einer Vielzahl von mikrokristallhaltigen Teilchen aus Glucopyranosylalditol(en), die in zufälliger Weise miteinander agglomeriert sind, bereitgestellt wird, wobei die Mikrokristalle eine Durchschnittsgrösse von weniger als 50 µm aufweisen.

2. Verfahren gemäss Anspruch 1, worin die gassuspendierten feinen Feststoffteilchen im wesentlichen aus Mikrokristallen aus einem oder mehreren der Glucopyranosylalditole bestehen.

3. Verfahren gemäss Anspruch 2, worin das Produkt im wesentlichen über seine gesamte Struktur aus einer Vielzahl von Mikrokristallen aus einem oder mehreren der in zufälliger Weise miteinander agglomerierten Glucopyranosylalditole besteht:

4. Verfahren gemäss mindestens einem der Ansprüche 1, 2 oder 3, worin das Glucopyranosylalditol 1-O-α-D-Glucopyranosyl-D-mannit (1,1-GPM) oder 6-O-α-D-Glucopyranosyl-D-sorbit (1,6-GPS) oder eine Mischung daraus ist, vorzugsweise eine äquimolare Mischung daraus (Isomalt).

5. Verfahren gemäss mindestens einem der vorhergehenden Ansprüche, worin die Flüssigkeit eine wässrige Lösung eines oder mehrerer Glucopyranosylalditol(s/e) mit einer Glucopyranosylalditol-Konzentration von etwa 30-80 Gew.%, vorzugsweise etwa 50-70 Gew.%, ist.

6. Verfahren gemäss mindestens einem der vorhergehenden Ansprüche, das die Erwärmung der Flüssigkeit auf eine Temperatur von etwa 45-95°C, vorzugsweise etwa 60-80°C, vor der Kontaktierung umfasst.

7. Verfahren gemäss mindestens einem der vorhergehenden Ansprüche, worin die Kontaktierung das Einsprühen der Flüssigkeit in Kontakt mit den gassuspendierten feinen Feststoffteilchen umfasst.

8. Verfahren gemäss mindestens einem der vorhergehenden Ansprüche, worin die Flüssigkeit eine geringe Menge eines Verdünnungsmittels, eines Bindemittels, eines aktiven Bestandteils und/oder anderer Süssstoffe als Glucopyranosylalditol enthält.

9. Verfahren gemäss mindestens einem der vorhergehenden Ansprüche, worin ein Sekundärspray einer anderen Flüssigkeit, die ein Verdünnungsmittel, ein Bindemittel, einen aktiven Bestandteil und/oder anderen Süssstoff enthält, gleichzeitig bereitgestellt wird.

10. Verfahren gemäss mindestens einem der vorhergehenden Ansprüche, worin die Entfernung des Lösungsmittels durchgeführt wird durch Einführung eines Trocknungsgases, wie beispielsweise Luft, das auf eine Temperatur von etwa 45-250°C, vorzugsweise etwa 70-160°C, erwärmt ist.

11. Verfahren gemäss Anspruch 10, worin das Lösungsmittel Wasser ist, und die Entfernung des Lösungsmittels liefert ein Glucopyranosylalditol-Material, das einen freien Feuchtigkeitsgehalt von etwa 0,1-2 %, vorzugsweise unter 1 %, aufweist, während das Glucopyranosylalditol-Material noch im gassuspendierten Zustand vorliegt.

12. Verfahren gemäss mindestens einem der vorhergehenden Ansprüche, worin die Konditionierung so gehalten wird, dass die Glucopyranosylalditol-Mikrokristallisation in der Zusammensetzung ablaufen kann.

13. Verfahren gemäss mindestens einem der vorhergehenden Ansprüche, worin sich die Glucopyranosylalditol-Zusammensetzung auf einem Förderband absetzen kann und darauf eine im wesentlichen kontinuierliche, agglomerierte, poröse Schicht mit einer Dicke von etwa 0,5-15 cm, vorzugsweise etwa 3-7 cm, bildet.

14. Verfahren gemäss Anspruch 13, worin die Konditionierung die Behandlung der Zusammensetzung in der agglomerierten Schicht mit einem Trocknungsgas mit einer Temperatur von etwa 40-100°C für eine Zeit von etwa 10-180 Minuten oder mehr einschliesst.

15. Verfahren gemäss Anspruch 14, worin die Konditionierung in mehreren aufeinanderfolgenden Schritten mit abnehmender Trocknungsgastemperatur durchgeführt wird.

16. Verfahren gemäss Anspruch 14 oder 15, das ferner die Abkühlung der konditionierten, agglomerierten Schicht unter Bereitstellung einer flachen porösen und brüchigen Platte, die mikrokristalline(s) Glucopyranosylalditol(e) enthält/enthalten, umfasst.

17. Verfahren gemäss Anspruch 15 oder 16, das die Ausübung von Reibwirkung auf die Platte zum Zerbrechen der agglomerierten Schicht und gegebenenfalls Zerkleinern des Produkts umfasst.

18. Verfahren gemäss mindestens einem der vorhergehenden Ansprüche, das ferner die Fraktionierung der mikrokristallinen Teilchen und Rezirkulierung zumindest eines Teils davon zur Bereitstellung eines Zufuhrmaterials aus den feinen Feststoffteilchen, die mikrokristalline(s) Glucopyranosylalditol(e) enthalten, umfasst.

19. Verfahren gemäss mindestens einem der vorhergehenden Ansprüche, worin etwa 30-70 %, vorzugsweise etwa 40-60 %, der Trockensubstanz aus einem Zufuhrmaterial aus festen mikrokristallinen Teilchen stammen.

20. Verfahren gemäss mindestens einem der Ansprüche 1 bis 11, worin die Feststoffteilchen in fluidisiertem Zustand gehalten werden, bis sie auf ein vorherbestimmtes Gewicht angewachsen sind.

21. Verfahren gemäss mindestens einem der vorhergehenden Ansprüche, das die Rezirkulierung von mikrokristallinen Glucopyranosylalditol-Teilchen mit einer mittleren Teilchengrösse von weniger als etwa 0,2 mm, vorzugsweise weniger als etwa 0,1 mm, umfasst.

22. Verfahren gemäss mindestens einem der vorhergehenden Ansprüche, worin das mikrokristalline Glucopyranosylalditol ein mikrokristallines Dihydrat von 1-O-α-D-Glucopyranosyl-D-mannit (1,1-GPM) oder wasserfreies 6-O-α-D-Glucopyranosyl-D-sorbit (1,6-GPS) oder eine Mischung daraus ist, vorzugsweise eine im wesentlichen äquimolare Mischung daraus (Isomalt).

23. Teilchenförmiges Glucopyranosylalditol-Produkt, worin jedes Produktteilchen eine Vielzahl von Mikrokristallen umfasst, die Teilchen aus einem oder mehreren Glucopyranosylalditolen enthalten, ausgewählt aus 1-O-α-D-Glucopyranosyl-D-mannit (1,1-GPM) und 6-O-α-D-Glucopyranosyl-D-sorbit (1,6-GPS) und gegebenenfalls 1-O-α-D-Glucopyranosyl-D-sorbit (1,1-GPS), die in zufälliger Weise miteinander agglomeriert sind, wobei die Mikrokristalle eine durchschnittliche Grösse von weniger als 50 µm aufweisen.

24. Glucopyranosylalditol-Produkt gemäss Anspruch 23, worin das Produktteilchen im wesentlichen über seine gesamte Struktur aus einer Vielzahl von Mikrokristallen aus einem oder mehreren der in zufälliger Weise miteinander agglomerierten Glucopyranosylalditole besteht.

25. Glucopyranosylalditol-Produkt gemäss Anspruch 23 oder 24, worin die Mikrokristalle aus 1-O-α-D-Glucopyranosyl-D-mannit-dihydrat oder wasserfreiem 6-O-α-D-Glucopyranosyl-D-sorbit oder einer Mischung daraus, vorzugsweise einer äquimolaren Mischung daraus (Isomalt), bestehen.

26. Glucopyranosylalditol-Produkt gemäss Anspruch 23 oder 24, worin die Mikrokristalle im wesentlichen aus wasserfreiem 6-O-α-D-Glucopyranosyl-D-sorbit bestehen.

27. Glucopyranosylalditol-Produkt gemäss Anspruch 23 oder 24, worin die Mikrokristalle im wesentlichen aus 1-O-α-D-Glucopyranosyl-D-mannit in Form des Dihydrats oder in einer teilweise oder vollständig dehydratisierten Form bestehen.

28. Glucopyranosylalditol-Produkt gemäss Anspruch 23 und 24, worin die Produktteilchen amorphes 1-O-α-D-Glucopyranosyl-D-sorbit enthalten.

29. Glucopyranosylalditol-Produkt gemäss Anspruch 23, das einen Gesamtgehalt an 1-O-α-D-Glucopyranosyl-Dmannit, 6-O-α-D-Glucopyranosyl-D-sorbit und 1-O-α-D-Glucopyranosyl-D-sorbit von mehr als 80 % aufweist, vorzugsweise mehr als 90 %, am meisten bevorzugt 98 % oder mehr.

30. Glucopyranosylalditol-Produkt gemäss Anspruch 24, worin die Produktteilchen hergestellt wurden durch Mikrokristallisation einer Flüssigkeit, die aufgelöste(s) Glucopyranosylalditol(e) enthält, zusammen mit feinen gassuspendierten Feststoffteilchen, die mikrokristalline(s) Glucopyranosylalditol(e) enthalten.

31. Glucopyranosylalditol-Produkt gemäss Anspruch 23, worin etwa 10-90 %, vorzugsweise etwa 30-80.%, weiter bevorzugt 40-60 %, der Trockensubstanz des Endprodukts aus einem Zufuhrmaterial aus festen mikrokristallinen Teilchen erhalten werden.

32. Glucopyranosylalditol-Produkt gemäss mindestens einem der vorhergehenden Ansprüche 23 bis 31, das Produktteilchen mit einer mittleren Teilchengrösse von etwa 0,1-2 mm, vorzugsweise etwa 0,15-0,4 mm, umfasst.

33. Glucopyranosylalditol-Produkt gemäss mindestens einem der vorhergehenden Ansprüche 23 bis 32, worin die Grösse der Mikrokristalle in jedem Teilchen im Durchschnitt unter 50 µm, vorzugsweise etwa 10 µm, liegt.

34. Glucopyranosylalditol-Produkt gemäss mindestens einem der vorhergehenden Ansprüche 23 bis 33, worin das Produktteilchen zusätzlich weitere Komponenten, wie beispielsweise Verdünner, Bindemittel, aktive Bestandteile und/oder andere Süssstoffe, in seiner Struktur integriert aufweist.

35. Verwendung des Mikrokristalle enthaltenden Glucopyranosylalditol-Produkts gemäss Anspruch 23 als Massesüssstoff für den vollständigen oder teilweisen Austausch von Sucrose.

36. Verwendung des Mikrokristalle enthaltenden Glucopyranosylalditol-Produkts gemäss Anspruch 23 in Konfekt, Backwaren, Cerealien, Desserts, Marmeladen, Getränken, Schokolade, Marzipan, Tischsüssmitteln, Kaugummi, Eiscreme und diätetischen Produkten sowie in pharmazeutischen Produkten.

37. Verwendung gemäss Anspruch 36, worin das Glucopyranosylalditol-Produkt in Hartsüsswaren verwendet wird.

38. Verwendung des Mikrokristalle enthaltenden Glucopyranosylalditol-Produkts gemäss Anspruch 23 in einem nicht-kariogenen Kaugummi.

39. Spezialsüssstoff, der mikrokristallhaltiges Glucopyranosylalditol gemäss Anspruch 23 umfasst.

40. Spezialsüssstoff gemäss Anspruch 39, der hauptsächlich zusammengesetzt ist aus mikrokristallinem Dihydrat von 1-O-α-D-Glucopyranosyl-D-mannit (1,1-GPM) oder wasserfreiem 6-O-α-D-Glucopyranosyl-D-sorbit (1,6-GPS) oder einer Mischung daraus, vorzugsweise einer im wesentlichen äquimolaren Mischung daraus (Isomalt).

41. Spezialsüssstoff gemäss Anspruch 39, der ferner andere Süssstoffe, ausgewählt aus Xylit, Maltitol, Lactitol und Sorbit, enthält.

42. Glucopyranosylalditol-Kaugummi, **dadurch gekennzeichnet, dass** es ein oder mehrere Glucopyranosylalditol(e) enthält, ausgewählt aus mikrokristallinem 1-O-α-D-Glucopyranosyl-D-mannitdihydrat, mikrokristallinem wasserfreien 6-O-α-D-Glucopyranosyl-D-sorbit und 1-O-α-D-Glucopyranosyl-Dsorbit, wobei die Mikrokristalle eine Durchschnittsgrösse von weniger als 50 µm aufweisen.

43. Kaugummi gemäss Anspruch 42, worin das mikrokristalline Glucopyranosylalditol hergestellt wurde durch Kontaktieren suspendierter mikrokristalliner Glucopyranosylalditol-Teilchen mit einer Glucopyranosylalditol-Lösung, Trocknen der resultierenden Zusammensetzung zur Bewirkung der Glucopyranosylalditol-Mikrokristallisation und Konditionierung der Zusammensetzung zur Bereitstellung eines Produkts, das im wesentlichen über seine gesamte Struktur aus einer Vielzahl von Mikrokristallen aus in zufälliger Weise miteinander agglomerierten Glucopyranosylalditol-Mikrokristallen besteht.

44. Glucopyranosylalditol-Hartsüsswaren, die **dadurch gekennzeichnet sind, dass** sie aus einem Glucopyranosylalditol-Produkt hergestellt sind, das nach dem Verfahren gemäss Anspruch 1 erhältlich ist und hergestellt wird durch Kontaktieren von gassuspendierten Teilchen, die ein oder mehrere mikrokristalline(s) Glucopyranosylalditol(e) umfassen, ausgewählt aus 1-O-α-D-Glucopyranosyl-Dmannit-dihydrat und wasserfreiem 6-O-α-D-Glucopyranosyl-D-sorbit, mit einer Lösung eines oder mehrerer Glucopyranosylalditole, ausgewählt aus 1-O-α-D-Glucopyranosyl-D-mannit, 6-O-α-D-Glucopyranosyl-D-sorbit und/oder 1-O-α-D-Glucopyranosyl-D-sorbit, Trocknen.der resultierenden Zusammensetzung zur Bewirkung der Glucopyranosylalditol-Mikrokristallisation und Konditionierung der Zusammensetzung zur Bereitstellung eines Produkts mit einer Vielzahl von Mikrokristallen, die Teilchen aus in zufälliger Weise miteinander agglomerierten Teilchen aus Glucopyranosylalditol(en) enthalten.

## Revendications

1. Procédé pour la cristallisation de glucopyranosylalditols comprenant :
- la mise en contact d'un liquide contenant un ou plusieurs glucopyranosylalditols dissous choisis dans le groupe constitué par le 1-O-α-D-glucopyranosyl-D-mannitol (1,1-GPM) et le 6-O-α-D-glucopyranosyl-D-sorbitol (1,6-GPS) et, de façon optionnelle, le 1-O-α-D-glucopyranosyl-D-sorbitol (1,1-GPS), avec des particules fines solides, contenant un ou plusieurs desdits glucopyranosylalditols, en suspension dans un gaz, pour mouiller les particules ;
- l'élimination substantielle du composant solvant dudit liquide et la soumission de la matière glucopyranosylalditol qui en résulte à une sédimentation pour que se forme une composition de matière essentiellement solide comprenant une multitude de microcristaux dudit ou des dits glucopyranosylalditols ; et
- la soumission de ladite composition de glucopyranosylalditol(s) à un conditionnement pendant une étape de séchage supplémentaire pour donner un produit avec des particules de glucopyranosylalditol(s) contenant une multitude de microcristaux agglomérés entre eux de manière aléatoire, lesdits microcristaux ayant une dimension moyenne inférieure à 50 µ.

2. Procédé selon la revendication 1, dans lequel les particules fines solides en suspension dans un gaz sont constituées essentiellement par des microcristaux d'un ou plusieurs desdits glucopyranosylalditols.

3. Procédé selon la revendication 2, dans lequel ledit produit est constitué essentiellement dans l'ensemble de sa structure par une multitude de microcristaux d'un ou plusieurs desdits glucopyranosylalditols agglomérés entre eux de manière aléatoire.

4. Procédé selon l'une quelconque des revendications 1, 2 ou 3, dans lequel ledit glucopyranosylalditol est le 1-O-α-D-glucopyranosyl-D-mannitol (1,1-GPM) ou le 6-O-α-D-glucopyranosyl-D-sorbitol (1,6-GPS) ou un mélange de ceux-ci, de préférence un mélange équimolaire de ceux-ci (isomalt).

5. Procédé selon l'une quelconque des revendications précédentes, dans lequel ledit liquide est une solution aqueuse d'un ou plusieurs glucopyranosylalditols ayant un taux de glucopyranosylalditol d'environ 30 à 80 % en masse, de préférence d'environ 50 à 70 % en masse.

6. Procédé selon l'une quelconque des revendications précédentes, comprenant le chauffage dudit liquide à une température d'environ 45 à 95°C, de préférence d'environ 60 à 80°C, avant ladite mise en contact.

7. Procédé selon l'une quelconque des revendications précédentes, dans laquelle ladite mise en contact comprend la pulvérisation dudit liquide avec lesdites particules fines solides en suspension dans un gaz.

8. Procédé selon l'une quelconque des revendications précédentes, dans lequel ledit liquide contient une portion mineure d'un excipient, d'un liant, d'un principe actif et/ou d'un édulcorant autre qu'un glucopyranosylalditol.

9. Procédé selon l'une quelconque des revendications précédentes, dans lequel on apporte simultanément une pulvérisation secondaire d'un autre liquide contenant un excipient, un liant, un principe actif et/ou un autre édulcorant.

10. Procédé selon l'une quelconque des revendications précédentes, dans lequel on réalise ladite élimination dudit solvant en introduisant un gaz de séchage tel que de l'air chauffé à une température d'environ 45 à 250°C, de préférence d'environ 70 à 160°C.

11. Procédé selon la revendication 10, dans lequel ledit solvant est de l'eau et ladite élimination du solvant donne une matière de glucopyranosylalditol séchée jusqu'à un taux d'humidité libre d'environ 0,1 à 2 %, de préférence inférieure à 1 %, tandis que ladite matière glucopyranosylalditol est toujours en suspension dans un gaz.

12. Procédé selon l'une quelconque des revendications précédentes, dans lequel ledit conditionnement est prolongé de telle façon que le glucopyranosylalditol forme des microcristaux dans ladite composition.

13. Procédé selon l'une quelconque des revendications précédentes, dans lequel on laisse se déposer ladite composition de glucopyranosylalditol sur un tapis roulant, et se former sur celui-ci une couche poreuse agglomérée substantiellement continue d'une épaisseur d'environ 0,5 à 15 cm, de préférence d'environ 3 à 7 cm.

14. Procédé selon la revendication 13, dans lequel ledit conditionnement inclut le traitement de ladite composition dans ladite couche agglomérée avec un gaz séchant ayant une température d'environ 40 à 100°C pendant une durée d'environ 10 à 180 mn ou plus.

15. Procédé selon la revendication 14, dans lequel ledit conditionnement est effectué en plusieurs étapes successives avec un gaz séchant à des températures décroissantes.

16. Procédé selon la revendication 14 ou 15, comprenant en outre le refroidissement de ladite couche agglomérée conditionnée pour obtenir une plaque plane poreuse et friable comprenant un ou plusieurs glucopyranosylalditol(s) microcristallin(s).

17. Procédé selon la revendication 15 ou 16, comprenant en outre la soumission de ladite plaque à une action de fragmentation de manière à diviser ladite couche agglomérée et éventuellement la fragmentation du produit.

18. Procédé selon l'une quelconque des revendications précédentes, comprenant en outre le fractionnement desdites particules microcristallines et le recyclage d'au moins une portion de celles-ci pour obtenir un courant d'alimentation de dites particules fines solides contenant un ou des glucopyranosylalditol(s) microcristallins.

19. Procédé selon l'une quelconque des revendications précédentes, dans lequel environ 30 à 70 %, de préférence environ 40 à 60 %, de la substance sèche provient d'un courant d'alimentation de particules microcristallines solides.

20. Procédé selon l'une quelconque des revendications 1 à 11, dans lequel lesdites particules solides sont maintenues à l'état fluidisé jusqu'à ce qu'elles aient atteint une masse prédéfinie.

21. Procédé selon l'une quelconque des revendications précédentes, comprenant le recyclage de particules microcristallines de glucopyranosylalditol ayant une dimension moyenne inférieure à environ 0,2 mm, de préférence inférieures à environ 0,1 mm.

22. Procédé selon l'une quelconque des revendications précédentes, dans lequel ledit glucopyranosylalditol microcristallin est le dihydrate microcristallin de 1-O-α-D-glucopyranosyl-D-mannitol (1,1-GPM) ou le 6-O-α-D-glucopyranosyl-D-sorbitol (1,6-GPS) anhydre, ou un mélange de ceux-ci, de préférence un mélange substantiellement équimolaire de ceux-ci (isomalt).

23. Produit de glucopyranosylalditol en particules dans lequel chaque particule de produit comprend des particules, d'un ou plusieurs glucopyranosylalditols choisis dans le groupe constitué par le 1-O-α-D-glucopyranosyl-D-mannitol (1,1-GPM) et le 6-O-α-D-glucopyranosyl-D-sorbitol (1,6-GPS) et, de façon optionnelle, le 1-O-α-D-glucopyranosyl-D-sorbitol (1,1-GPS), contenant une multitude de microcristaux agglomérés ensemble de façon aléatoire, lesdits microcristaux ayant une dimension moyenne inférieure à 50 µ.

24. Produit de glucopyranosylalditol selon la revendication 23, lesdites particules du produit étant constituées essentiellement dans l'ensemble de la structure par une multitude de microcristaux d'un ou plusieurs desdits glucopyranosylalditols agglomérés entre eux de manière aléatoire.

25. Produit de glucopyranosylalditol selon la revendication 23 ou 24, où lesdits microcristaux sont constitués par du dihydrate de 1-O-α-D-glucopyranosyl-D-mannitol ou du 6-O-α-D-glucopyranosyl-D-sorbitol anhydre, ou un mélange de ceux-ci, de préférence un mélange équimolaire de ceux-ci (isomalt).

26. Produit de glucopyranosylalditol selon la revendication 23 ou 24, où lesdits microcristaux sont constitués essentiellement par du 6-O-α-D-glucopyranosyl-D-sorbitol anhydre.

27. Produit de glucopyranosylalditol selon la revendication 23 ou 24, où lesdits microcristaux sont constitués essentiellement par du 1-O-α-D-glucopyranosyl-D-mannitol sous la forme dihydratée ou sous forme partiellement ou totalement déshydratée.

28. Produit de glucopyranosylalditol selon la revendication 23 ou 24, où lesdites particules de produit contiennent du 1-O-α-D-glucopyranosyl-D-sorbitol amorphe.

29. Produit de glucopyranosylalditol selon la revendication 23, ayant au total un taux de 1-O-α-D-glucopyranosyl-D-mannitol, de 6-O-α-D-glucopyranosyl-D-sorbitol et de 1-O-α-D-glucopyranosyl-D-sorbitol supérieur à 80 %, de préférence supérieur à 90 %, le plus préférablement égal ou supérieur à 98 %.

30. Produit de glucopyranosylalditol selon la revendication 24, lesdites particules du produit ayant été produites par la microcristallisation d'un liquide contenant un ou des glucopyranosylalditols dissous en combinaison avec des particules solides fines, contenant un ou des glucopyranosylalditols microcristallins, en suspension dans un gaz.

31. Produit de glucopyranosylalditol selon la revendication 23, où environ 10 à 90 %, de préférence environ 30 à 80 %, le plus préférablement 40 à 60 %, de la substance sèche du produit final provient d'un courant d'alimentation de particules microcristallines solides.

32. Produit de glucopyranosylalditol selon l'une quelconque des revendications précédentes 23 à 31, comprenant des particules de produit ayant une dimension moyenne d'environ 0,1 à 2 mm, de préférence d'environ 0,15 à 0,4 mm.

33. Produit de glucopyranosylalditol selon l'une quelconque des revendications précédentes 23 à 32, où la dimension des microcristaux dans chaque particule est en moyenne inférieure à 50 µ, de préférence d'environ 10 µ.

34. Produit de glucopyranosylalditol selon l'une quelconque des revendications précédentes 23 à 33, où lesdites particules de produits contiennent en outre, intégrées dans leur structure, des composants supplémentaires tels que des excipients, des liants, des principes actifs et/ou d'autres édulcorants.

35. Utilisation du produit de glucopyranosylalditol contenant des microcristaux selon la revendication 23 en tant qu'édulcorant courant pour le remplacement total ou partiel du saccharose.

36. Utilisation du produit de glucopyranosylalditol contenant des microcristaux selon la revendication 23 en confiserie, dans les produits de boulangerie, les céréales, les desserts, les confitures, les boissons, le chocolat, la pâte d'amandes, les édulcorants de table, le chewing-gum, les crèmes glacées et les produits diététiques, ainsi que dans les produits pharmaceutiques.

37. Utilisation selon la revendication 36, ledit produit de glucopyranosylalditol étant utilisé dans des bonbons durs.

38. Utilisation du produit de glucopyranosylalditol contenant des microcristaux selon la revendication 23 dans un chewing-gum non-cariogène.

39. Edulcorant spécial, comprenant le produit de glucopyranosylalditol contenant des microcristaux selon la revendication 23.

40. Edulcorant spécial selon la revendication 39, composé principalement de dihydrate microcristallin de 1-O-α-D-glucopyranosyl-D-mannitol (1,1-GPM) ou de 6-O-α-D-glucopyranosyl-D-sorbitol (1,6-GPS) anhydre, ou d'un mélange de ceux-ci, de préférence d'un mélange substantiellement équimolaire de ceux-ci (isomalt).

41. Edulcorant spécial selon la revendication 39, contenant en outre d'autres édulcorants choisis dans le groupe constitué par le xylitol, le maltitol, le lactitol et le sorbitol.

42. Chewing-gum avec glucopyranosylalditol, **caractérisé en ce qu'**il contient un ou plusieurs glucopyranosylalditols choisis dans le groupe constitué par le dihydrate de 1-O-α-D-glucopyranosyl-D-mannitol microcristallin, le 6-O-α-D-glucopyranosyl-D-sorbitol anhydre microcristallin et le 1-O-α-D-glucopyranosyl-D-sorbitol, lesdits microcristaux ayant une dimension moyenne inférieure à 50 µ.

43. Chewing-gum selon la revendication 42, ledit glucopyranosylalditol microcristallin ayant été produit par la mise en contact de particules microcristallines de glucopyranosylalditol en suspension avec une solution de glucopyranosylalditol, le séchage de la composition obtenue pour que se produise la microcristallisation du glucopyranosylalditol, et le conditionnement de ladite composition pour obtenir un produit constitué essentiellement dans l'ensemble de la structure par une multitude de microcristaux de glucopyranosylalditol agglomérés entre eux de manière aléatoire.

44. Bonbon dur avec glucopyranosylalditol, **caractérisé en ce qu'**il est fabriqué à partir d'un produit de glucopyranosylalditol pouvant être obtenu conformément au procédé de la revendication 1 et produit par la mise en contact de particules, en suspension dans un gaz, comprenant un ou plusieurs glucopyranosylalditols microcristallins choisis dans le groupe constitué par le dihydrate de 1-O-α-D-glucopyranosyl-D-mannitol et le 6-O-α-D-glucopyranosyl-D-sorbitol anhydre avec une solution d'un ou plusieurs glucopyranosylalditols choisis dans le groupe constitué par le 1-O-α-D-glucopyranosyl-D-mannitol, le 6-O-α-D-glucopyranosyl-D-sorbitol et/ou le 1-O-α-D-glucopyranosyl-D-sorbitol, le séchage de la composition obtenue pour que se produise la microcristallisation du glucopyranosylalditol et le conditionnement de ladite composition pour obtenir un produit constitué de particules de glucopyranosylalditol contenant une multitude de microcristaux agglomérés entre eux de manière aléatoire.
